# EUROPÄISCHE PATENTSCHRIFT

(11) **EP 1 406 587 B1**
(45) Veröffentlichungstag und Bekanntmachung des Hinweises auf die Patenterteilung: **03.05.2006**
(21) Anmeldenummer: 02762352.9
(22) Anmeldetag: 12.07.2002
(51) Int. Cl.: A61K 8/02, A61K 8/34, A61K 8/33, A61K 8/37, A61K 8/362, A61K 8/92, A61Q 19/00

(54) **ZUSAMMENSETZUNGEN AUF WACHSBASIS UND VERWENDUNG ALS KÖRPERPFLEGEMITTEL**
WAX-BASED COMPOSITIONS
COMPOSITION A BASE DE CIRE

(30) Priorität: 13.07.2001 DE 10133399
(43) Veröffentlichungstag der Anmeldung: 14.04.2004
(60) Teilanmeldung: 06003669.6
(73) Patentinhaber: Cognis IP Management GmbH, 40589 Düsseldorf (DE)
(72) Erfinder: ANSMANN, Achim, 40699 Erkrath (DE); ISSBERNER, Ulrich, 41569 Rommerskirchen (DE); BRÜNING, Stefan, 40233 Düsseldorf (DE); JACKWERTH, Bettina, 40764 Langenfeld (DE); HOFFMANN, Daniele, 40597 Düsseldorf (DE)
(86) Internationale Anmeldenummer: PCT/EP2002/007787
(87) Internationale Veröffentlichungsnummer: WO 2003/005981

(56) Entgegenhaltungen:
- EP-A- 0 691 125
- WO-A-01/39766
- WO-A-01/52806
- WO-A-01/56396
- WO-A-95/35412
- WO-A-99/17714
- DE-A- 19 828 020

## Beschreibung

### Gebiet der Erfindung

Gegenstand der Erfindung sind spezielle Zusammensetzungen auf Wachsbasis, die als Grundlage für kosmetische Mittel verwendet werden können sowie insbesondere zur Imprägnierung und Benetzung von Gebrauchs- und Hygienetüchem, die zur Körperreinigung und -pflege eingesetzt werden.

### Stand der Technik

Unter dem Oberbegriff "Papier" werden ca. 3000 verschiedene Sorten und Artikel verstanden, deren Beschaffenheit und Anwendungsgebiete sich zum Teil erheblich unterscheiden können. Zur Herstellung von Papier benötigt man eine Reihe von Zusatzstoffen, von denen Füllstoffe (z.B. Kreide oder Kaolin) und Bindemittel (z.B. Stärke) zu den wichtigsten zählen. Für den Bereich der Tissue- und Hygienepapiere, die in engeren Kontakt mit der menschlichen Haut gebracht werden, besteht ein besonderes Bedürfnis nach einem angenehmen Weichgriff, der dem Papier üblicherweise durch eine sorgfältige Auswahl der Faserstoffe und insbesondere einen hohen Anteil an frischem Holzschliff oder Cellulose verliehen wird. Im Hinblick auf die Wirtschaftlichkeit der Papierherstellung sowie aus ökologischer Sicht, ist es jedoch wünschenswert, möglichst hohe Anteile an qualitativ minderwertigerem Altpapier mitzuverwenden. Dies hat jedoch zur Folge, daß der Weichgriff des Papiers signifikant verschlechtert wird, was von den Anwendem als störend empfunden wird und insbesondere bei häufigem Gebrauch auch zu Hautimtationen führen kann.

In der Vergangenheit hat es daher nicht an Versuchen gemangelt, Papier durch Tränken, Beschichten oder andere Oberflächenbehandlung so zu modifizieren, daß eine angenehmere Sensorik resultiert. Hierfür müssen spezielle Lotionen und Emulsionen entwickelt werden, die sich einerseits leicht auf das Papier auftragen lassen, anderseits dessen Struktur nicht negativ beeinflussen. Um den Weichgriff zu verbessem, werden häufig Niotenside oder eine Kombination aus Nio- und Aniontensiden verwendet. Auch Polysiloxane und kationische Polymere werden für diesen Zweck eingesetzt.

Gegenstand der internationalen Patentanmeldung **WO 95/35411** sind Tissuepapiere, die mit Avivagemitteln beschichtet werden, welche 20 bis 80 Gew.-% eines wasserfreien Emolliens (Mineralöle, Fettsäureester, Fettalkoholethoxylate, Fettsäureethoxylate, Fettalkohole und deren Mischungen), 5 bis 95 Gew.-% eines das Emolliens "immobilisierenden Agens" (Fettalkohole, Fettsäuren oder Fettalkoholethoxylate mit jeweils 12 bis 22 Kohlenstoffatomen im Fettrest) sowie 1 bis 50 Gew.-% Tenside mit einem HLB-Wert von vorzugsweise 4 bis 20 enthalten. Die in der Schrift aufgeführten Ausführungsbeispiele enthalten als Emolliens ausnahmslos Petrolatum. Die internationale Patentanmeldung **WO 95/35412** offenbart ähnliche Tissuepapiere, wobei als Softener wasserfreie Mischungen von (a) Mineralölen, (b) Fettalkoholen oder Fettsäuren und (c) Fettalkoholethoxylaten zum Einsatz kommen. Gegenstand der internationalen Patentanmeldung **WO 95/16824** sind Avivagemittel für Tissuepapiere, die Mineralöl, Fettalkoholethoxylate und nichtionische Tenside (Sorbitanester, Glucamide) enthalten. Des weiteren werden in der internationalen Patentanmeldung **WO 97/30216** (Kaysersberg) flüssige Avivagemittel für Papiertaschentücher auf Basis von langkettigen, gesättigten Fettalkoholen und Wachsestem mit insgesamt wenigstens 24 Kohlenstoffatomen beschrieben, die einen sehr hohen Wasseranteil enthalten. Die Patentschrift **DE 33 09 530** beschreibt hygienische Absorptionsvorlagen, die mit Glyceriden und/oder Partialglyceriden der Kokosfettsäuren belegt sind. Beschichtungen für Hygieneprodukte werden auch in R. E. Mathis, **Nonwovens World 1999**, Seiten 59 - 65 beschrieben.

Vom anwendungstechnischen Standpunkt ist insbesondere die Sensorik der behandelten Papiere und Tissues nach wie vor verbesserungswürdig. Die gegenwärtig verwendeten Beschichtungen hinterlassen oft ein zu fettiges Hautgefühl und zeichnen sich zum Teil durch eine zu langsame Wirkstofffreigabe aus. Insbesondere auf dem Gebiet der Baby-Hygiene ist eine effektive WirkstoffFreisetzung, eine verbesserte Pflegeleistung und Sensorik von großer Bedeutung.

Der Erfindung lag die Aufgabe zugrunde, Zusammensetzungen zur Beschichtung von Tissuepapieren und Wet Wipes zur Verfügung zu stellen, die sich durch eine verbesserte Sensorik auszeichnen, insbesondere ein weniger fettiges Hautgefühl. Ein weiterer Teilaspekt der Aufgabe war es, Zusammensetzungen zur Verfügung zu stellen, die im verflüssigten Zustand auf die Papiere appliziert werden können und eine wässrige Nachbehandlung der Papiere erlauben, ohne daß sich die Zusammensetzungen lösen. Ein weiterer Aspekt war es, Zusammensetzungen zu entwickeln, die nach Applikation auf Papieren mit wässriger Nachbehandlung der Papiere/Wipes lagerstabil sind und sich nicht vermischen, also bei längerer Lagerung keine Emulsionen bilden. Ein weiterer Teilaspekt der Aufgabe war es, Zusammensetzungen zu entwickeln, die eine effiziente Wirkstofffreisetzung gewährleisten. Die beschichteten Wipes sollten ausgezeichnete pflegende Eigenschaften aufweisen und sich durch besondere Milde und Hautverträglichkeit auszeichnen. Des weiteren sollten nur leicht biologisch abbaubare Hilfsstoffe Verwendung finden und die Zubereitungen trotz des sehr geringen Wasseranteils leicht in das Tissue eindringen, sich homogen verteilen, und leicht verarbeilbar sein.

### Beschrelbung der Erfindung

Es wurde gefunden, daß wachsartige Zubereitungen mit einem Gehalt an ganz speziellen Öl- oder Wachskörpem sowie einem sehr geringen Wassergehalt ausgezeichnete sensorische und pflegende Eigenschaften aufweisen, sehr leicht applizierbar sind und sich durch besondere Milde auszeichnen.

Gegenstand der Erfindung sind daher Zusammensetzung auf Wachsbasis mit einem Schmelzpunkt oberhalb 25 °C enthaltend
(a) wenigstens eine Öl- oder Wachskomponente ausgewählt aus C₁₄-C₃₀₋Dialkyl(en)carbonaten,
(b) weniger als 10 Gew.-% Wasser.

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30° C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar sind wachsartige Zusammensetzungen, die sich durch einen Schmelzpunkt auszeichnen, der oberhalb von 25 °C liegt.

Durch den Gehalt ganz spezieller Öl- oder Wachskomponenten, die ausgewählt sind aus den C₁₄-C₃₀ Dialkyl(en)carbonaten, läßt sich insbesondere die Sensorik solcher wachsartiger Zusammensetzungen optimieren, so daß sie als weniger fettig empfunden werden und ein eher trockenes Hautgefühl vermitbeln, aber dennoch ausgezeichnete pflegende Eigenschaften aufweisen. Die erfindungsgemäßen Zusammensetzungen enthalten weniger als 10 Gew.-% Wasser, vorzugsweise liegt der Wassergehalt bei weniger als 6 Gew.-%, und insbesondere weniger als 3 Gew.-%. In einer besonders bevorzugten Ausführungsform sind die Zusammensetzungen wasserfrei. Unter wasserfrei im Sinne der Erfindung ist zu verstehen, daß die Zusammensetzungen lediglich rohstonbedingt einen geringen Wasseranteil enthalten können, daß aber kein zusätzliches Wasser zugegeben wird. Beim Verarbeitungsprozeß und Applikation der Zusammensetzung auf die Wipes erlaubt dies einen Nachbehandiungsschritt der Wipes mit wässrig/tensidischen Lösungen, ohne daß sich die Zusammensetzung ablöst.

Die erfindungsgemäßen Zusammensetzungen lassen sich ausschließlich aus C₁₄-C₃₀ Dialkyl(en)carbonaten formuliere enthalten aber vorzugsweise - und je nach Anfrderungsprofil - weitere wachsartige Lipidkomponenten und Öle. Wesentlich ist daß der Schmelzbereich der Gesamtzusammensetzung oberhalb 25° liegt, d.h. daß sich die Zusammensetzung oberhalb dieser Temperatur im verflüssigten Zustand auf die Papiere applizieren läßt Es können also erfindungsgemäß auch Rüssige Dialkyl(en)ether, Dialkyl(en)carbonate, Dicarbonsäuren oder Hydroxyfettalkohole eingesetzt werden, solange die Gesamtzusammensetzung den geförderten Schmelzpunkt von höher als 25 °C aufweist. Eine bevorzugte Ausführungsform ist dadurch gekennzeichnet daß sie im Bereich von ca. 30 - 45°C, insbesondere bei 32 - 40°C schmitzt. Hierdurch wird gewährleistet, daß sich die Zusammensetzung nach Beschichtung des Papiers wieder verfestigt, eine Nachbehandlung der Wipes mit wässrig/tensidischen Lösungen und/oder Lotionen hierdurch erleichtert wird und ein weicher, nicht-spröder Film auf den Wipes hinterlassen wird. Mit derartigen Zusammensetzungen beschichtete Tücher sind besonders lagerstabil, eine Vermischung der Phasen wird vermieden. Des weiteren wird gewährleistet, daß die Zusammensetzung erst bei Applikation der Tücher auf der Haut wieder schmilzt und erst dann mit der wässrigen Phase emulgiert.

Erfindungsgemäß besonders vorteilhaft sind Zusammensetzung mit einem Penetrationswert von 0,2- 3,0 mm (Apparatur: Petrotester PNR 10, Mikrokonus, 5 sec., Temperatur 20 °C).

Die erfindungsgemäßen Zubereitungen enthalten wachsartige, lineare oder verzweigte, gesättigte oder ungesättigte C₁₄-C₃₀-Dialkyl(en)carbonate. Besonders bevorzugt sind C₁₆-C₂₄-Dialkyl(en)carbonate und unter diesen gesättigte, unverzweigte C₁₆-C₂₂₋Dialkylcarbonate. Besonders bevorzugt geeignet Ist Distearylcarbonat. Aber auch flüssige Dialkyl(en)carbonate, wie z. B. Dihexyl-, Dioctyl-, Di-(2-ethylhexyl)- oder Dioleylcarbonat, sind efindungsgemäß einsetzbar, solange die Gesamtzusammensetzung den geforderten Schmelzpunkt aufweist. Die Verbindungen lassen sich durch Umesterung von Dimethyl- oder Diethylcarbonat mit den entsprechenden Hydroxyverbindungen nach Verfahren des Standes der Technik hersteilen; eine Übersicht hierzu findet sich in Chem.Rev. 96, 951 (1996). Typische Beispiele für Dialkyl(en)carbonate sind Umesterungsprodukte von Dimethyl-und/oder Diethylcarbonat mit Capronalkchol, Caprylalkohol, 2-Ethylhexylaikohol, Caprinalkohol, Laurylalkohol. Myristylalkohol, Cetylalkohol, Palmoleylalkohol, Stearylalkohol, lsostearylaikohol, Etaidyialkohol, Petroselinylalkohol, Linolylalkohol, Linolenylalkohol, Oleylalkohol, Rizinolalkohol, Elaeostearylalkohol, Arachidylalkohol, Gadoleylalkohol, Behenylalkohol, Erucylalkohol und Brassidylalkohol, Guerbehalkoholen, sowie deren technische Mischungen, die z.B. bei der Hochdruckhydrierung von technischen Methylestem auf Basis von Fetten und Ölen anfallen. Bevorzugt geeignet sind bei 25 °C feste Dialkyl(en)carbonate. in einer Ausfürhungsform der Erfindung enthalten die zusammenserzungen zusätzlich Dialkyl (en) ether, Dicarbonsäuren oder Hydroxy-fettsäuren oder ein beliebiges gemisch dieser substanzen.

Die Dialkyl(en)ether können symmetrisch oder unsymmetrisch, verzweigt oder unverzweigt, gesättigt oder ungesättigt sein. Erfindungsgemäß bevorzugt geeignet sind wachsartige, gesättigte C₁₆-C₃₀₋Dialkylether, insbesondere C₁₆-C₂₄-Dialkylether. Besonders bevorzugt sind C₁₆-C₂₀-Dialkylether, und insbesondere bevorzugt geeignet ist Distearylether und Dibehenylether. Erfindungsgemäß können auch kürzerkettige Dialkylether eingesetzt werden, wie beispielsweise Di-n-octylether, Di-(2-ethylhexyl)-ether, Laurylmethylether oder Octybutylether, Didodacylether, solange die Gesamtzusammensetzung den geforderten Schmetzpunk hat. Die Verbindungen lassen sich aus Fettalkoholen In Gegenwart saurer Katalysatoren nach allgemein bekannten Verfahren des Standes der Technik herstellen, z. B. DE 19511 668 A1 und DE 198 31705 A1 sowie DE 199 43 585. Typische Beispiele für derartige Ether sind Produkte, die durch Veretherung von Cepronalkohol, Caprylalkohol, 2-Ethylhexylalkohol, Caprinalkohol, Laurylalkohol, Myristylalkohol, Cetylalkohol, Palmoleylalkohol, Stearylalkohol, Isostearylalkohol, Elaidylalkohol, Petroselinylalkohol, Linolylalkohol, Linolenylalkohol, 0leylalkohol, Rizinolalkohol, Elaeostearylalkohol, Arachldylalkohol, Gadoleylalkohol, Behenylalkohol, Erucylalkohol und Brassidylalkohol, Guerbetalkoholen, sowie deren technische Mischungen, die z.B. bei der Hochdruckhydrierung von technischen Methylestem auf Basis von Fetten und Ölen anfallen, gewonnenen werden. Bevorzugt geeignet sind bei 25 °C feste Dialkyl(en)ether.

Als Dicarbonsäuren lassen sich erfindungsgemäß C₉-C₃₄-Dicarbonsäuren einsetzen. Hierzu zählen z. B. Octadecandisäure, Tetratridecansäure, etc. Erfindungsgemäß bevorzugt geeignet ist Azelainsäure, eine C₉-Dicarbonsäure.

Unter den Hydroxyfettalkoholen sind gesättigte oder ungesättigte, verzweigte oder unverzweigte Verbindungen geeignet. C₁₂-C₃₀-Fettalkohole sind bevorzugt geeignet, wobei die Stellung des Hydroxy-Substituenten vom Syntheseweg und den eingesetzten Edukten abhängt. Hierzu zählen z. B. 1.10-Decandiol (Speziol® 10/2), 1,2-Hexadecandiol, 12-Hydroxystearylalkohol oder Hydroxy-Guerbetalkohole. Erfindungsgemäß bevorzugt geeignet sind bei 25 °C feste Hydroxyfettalkohole, obgleich auch flüssige einsetzbar sind, solange die Gesamtzusammensetzung den geforderten Schmelzpunkt aufweist. Besonders bevorzugt geeignet ist 12-Hydroxystearylalkohol, der von der Cognis France S.A. unter der Bezeichnung Speziol® 18/2 vermarktet wird. 1,2 Hexadecandiol erhält man durch Ringöffnung des entsprechenden α-Epoxids.

Die Dialkylether, Dialkylcarbonate und Dicarbonsäuren sowie Hydroxyalkohole sind bezogen auf die Gesamtzusammensetzung vorzugsweise in einer Menge von insgesamt 1-30 Gew.-%, besonders bevorzugt 1 - 20 Gew.-% und insbesondere 1-10 Gew.-% enthalten.

Die erfindungsgemäßen Zusammensetzungen sind praktisch geruchsfrei, ökotoxikologisch unbedenklich und leicht biologisch abbaubar. Sie eignen sich als fetthaltige, milde kosmetische Mittel und können auch als Basis in alle kosmetischen Mittel zur Körperpflege und -reinigung wie Cremes, Lotionen, sprühbare Emulsionen, Sonnenschutzmittel, Antitranspirantien, Flüssig- und Stückseifen etc. eingearbeitet werden. Sie lassen sich als Pflegekomponente auf Tissues, Papiere, Wipes, Schwämme, Puffs, Pflaster und Bandagen applizieren, die ihren Einsatz im Bereich der Hygiene und Pflege finden (Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes).

Die erfindungsgemäßen Zusammensetzungen können durch Einarbeitung weiterer Hilfs- und Zusatzstoffe als Pulver, Tabletten, in poröser Form, als Granulate, in verkapselter oder mikroverkapselter Form angeboten werden.

### Weitere wachsartige Lipidkomponenten

In einer weiteren bevorzugten Ausführungsform enthält die Zusammensetzung weitere wachsartige Lipidkomponenten. Durch den Zusatz weiterer wachsartiger Lipidkomponenten läst sich die Sensorik der Zusammensetzung sowie die Stabilität der Zusammensetzung nach Applikation auf die Papiere weiter optimieren und dem Anforderungsprofil anpassen.

Als weitere Lipidkomponenten (Definition vgl.: **CD Römpp Chemie Lexikon - Version 1.0**, **Stuttgart/New York: Georg Thieme Verlag 1995**) können erfindungsgemäß alle Fette und fettähnlichen Substanzen mit wachsartiger Konsistenz eingesetzt werden. Hierzu gehören u.a. Fette (Triglyceride), Mono- und Diglyceride, Wachse, Fett- und Wachsalkohole, Fettsäuren, Ester von Fettalkoholen sowie Fettsäureamide oder beliebige Gemische dieser Substanzen. Sie können in den erfindungsgemäßen Zusammensetzungen in einer Gesamtmenge von 0,1 - 90 Gew.-% enthalten sein. Bevorzugt sind Mengen von 5 - 65 Gew.-% und insbesondere 20 - 65 Gew.-% bezogen auf die Gesamtzusammensetzung.

### Fette

Unter Fetten versteht man Triacylglycerine, also die Dreifachester von Fettsäuren mit Glycerin. Unter den Triacylglycerinen sind jene als Lipidkomponente bevorzugt, die sich im Bereich von 30 - 45 °C, insbesondere 32- 40 °C verflüssigen, die also einen der Gesamtzusammensetzung vergleichbaren Schmelzbereich aufweisen. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind sogenannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkemöl, Leinöl, Mandelöl, Maisöl, Olivenöl, Sesamöl, Kakaobutter und Kokosfett.

Geeignet sind u.a. die Dreifachester von Glycerin mit C₁₂-C₆₀-Fettsäuren und insbesondere C₁₂-C₃₆₋Fettsäuren. Hierzu zählt gehärtetes Rizinusöl, ein Dreifachester aus Glycerin und einer Hydroxystearinsäure, der beispielsweise unter der Bezeichnung Cutina® HR im Handel ist. Ebenso geeignet sind Glycerintristearat, Glycerintribehenat (z. B. Syncrowax® HRC), Glycerintripalmitat oder die unter der Bezeichnung Syncrowax® HGLC bekannten Triglycerid-Gemische, mit der Vorgabe, daß der Schmelzpunkt der Gesamtzusammensetzung oberhalb von 25 °C, und vorzugsweise bei 30 - 45 °C liegt.

Als Lipidkomponenten sind neben den Triglyceriden auch Mono- und Diglyceride bzw. Mischungen der Glyceride einsetzbar. Zu den erfindungsgemäß bevorzugten Glyceridgemischen zählen die von der Cognis Deutschland GmbH vermarkteten Produkte Novata® AB und Novata® B (Gemisch aus C₁₂-C₁₈₋Mono-, Di- und Triglyceriden) sowie Cutina® MD oder Cutina® GMS (Glycerylstearat). Eine bevorzugte Ausführungsform der erfindungsgemäßen Zusammensetzung enthält als weitere wachsartige Lipidkomponente wenigstens ein Fettsäureglycerid aus der Gruppe der Mono-, Di- oder Triester von Glycerin mit Fettsäuren oder einem beliebigen Gemisch davon. Das Glycerid(gemisch) ist üblicherweise in einer Menge von weniger als 80 Gew.-% bezogen auf die Gesamtzusammensetzung enthalten, vorzugsweise weniger als 70 Gew.-% und besonders bevorzugt weniger als 60 Gew.-%.

Mischester sowie Mischungen aus Mono-, Di- und Triglyceriden sind erfindungsgemäß bevorzugt geeignet, da sie eine geringere Neigung zur Kristallisation zeigen und somit die Performance der erfindungsgemäßen Zusammensetzung verbessem.

### Fettalkohole und Fettsäuren

Zu den erfindungsgemäß einsetzbaren Fettalkoholen zählen die-C₁₂-C₅₀-Fettalkohole, insbesondere die C₁₂-C₂₄-Fettalkohole, die aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohot, Brassidylalkohol, Lignocerylalkohol, Cerylalkohol oder Myricylalkohol sowie Guerbetalkohole. Erfindungsgemäß bevorzugt sind gesättigte, verzweigte oder unverzweigte Fettalkohole. Aber auch ungesättigte, verzweigte oder unverzweigte Fettalkohole können erfindungsgemäß verwendet werden, solange die Gesamtzusammensetzung den geforderten Schmelzpunkt aufweist. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkemöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole®) oder die teilweise verzweigten Alkohole aus der Oxosynthese (Dobanole®) verwendet werden. Eine einer bevorzugten Ausführungsform der erfindungsgemäßen Zusammensetzung enthält als weitere wachsartige Lipidkomponente wenigstens einen Fettalkohol. Erfindungsgemäß bevorzugt geeignet sind C₁₄-C₁₈-Fettalkohole, die beispielsweise von der Cognis Deutschland GmbH unter der Bezeichnung Lanette® 16 (C₁₆-Alkohol), Lanette® 14 (C₁₄-Alkohol), Lanette® O (C₁₆/C₁₈-Alkohol) und Lanette® 22 (C₁₈/C₂₂-Alkohol) vermarktet werden. Ebenso bevorzugt geeignet ist ein C₁₆/C₁₈-Guerbetalkohol, der von der Cognis Deutschland GmbH unter der Bezeichnung Eutanol® G 32/36 im Handel ist. Fettalkohole verleihen den Zusammensetzungen ein trockeneres Hautgefühl als Triglyceride und sind daher bevorzugt geeignet.

Als zusätzliche wachsartige Lipidkomponenten können auch C₁₄-C₄₀-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

### Wachse

Erfindungsgemäß verwendbar sind beispielsweise natürliche pflanzliche Wachse, wie Candelillawachs, Camaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Ester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispielshaft sind hier zu erwähnen die C₁₆-C₄₀-Alkylstearate, C₂₀-C₄₀-Alkylstearate (z. B. Kesterwachs® K82H), C₂₀₋C₄₀-Dialkylester von Dimersäuren, C₁₈-C₃₈-Alkylhydroxystearoylstearate oder C₂₀-C₄₀-Alkylerucate. Ferner sind C₃₀-C₅₀-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat vorteilhaft einsetzbar. Für Wipes für die Hautpflege ist u.a. Myristyllactat (Cegesoft® C17) besonders gut geeignet, da es ein gutes Bindevermögen zur Haut aufweist. Auch Siliconwachse sind gegebenenfalls vorteilhaft.

Als weitere Konsistenzgeber können ggf. geringe Mengen an Alkalimetall- und Erdalkalimetall- sowie Aluminiumsalze von C₁₂-C₂₄-Fettsäuren oder C₁₂-C₂₄-Hydroxyfettsäuren eingesetzt werden, wobei Calcium-, Magnesium-, Aluminium- und insbesondere Zinkstearat bevorzugt ist.

### Ölkörper

In einer weiteren bevorzugten Ausführungsform enthält die erfindungsgemäße Zusammensetzung wenigstens einen Ölkörper. Unter Ölkörpem sind erfindungsgemäß bei 20 °C flüssige, mit Wasser bei 25 °C nicht mischbare Stoffe oder Gemische von Stoffen zu verstehen. Hierzu gehören alle Ölkörper die nicht unter die in Anspruch 1 genannten Dialkyl(en)ether, Dialkyl(en)carbonate, Dicarbonsäure oder Hydroxyfettalkohole fallen, also z. B. bei 20 °C flüssige Glyceride, Kohlenwasserstoffe, Silikonöle, Esteröle oder beliebige Gemische davon. Die Ölkörper sind in den erfindungsgemäßen Zusammensetzungen üblicherweise in Menge von weniger als 40 Gew.-%, bevorzugt in Mengen von 1-15 und insbesondere in Mengen von 2 - 10 Gew.-% bezogen auf die Gesamtzusammensetzung enthalten. Die Menge der eingearbeiteten Öle wird durch die Maßgabe limitiert, daß der Schmelzpunkt der gesamten Zusammensetzung oberhalb 25 °C liegen muß. Derartige Optimierungen gehören zu Routine-Optimierungen des Fachmanns.

Zu den als Ölkörper erfindungsgemäß einsetzbaren Glyceriden zählen bei 20°C flüssige Fettsäureester des Glycerins, die natürlicher (tierischer und pflanzlicher) oder synthetischer Herkunft sein können. Man unterscheidet zwischen Mono-, Di- und Triglyceriden. Es handelt sich um bekannte Stoffe, die nach einschlägigen Verfahren der präparativen organischen Chemie hergestellt werden können. Synthetisch hergestellte Glyceride sind üblicherweise Mischungen von Mono-, Di- und Triglyceriden, die durch Umesterung der entsprechenden Triglyceride mit Glycerin oder durch gezielte Veresterung von Fettsäuren erhalten werden. Als Fettsäure sind erfindungsgemäß C₆-C₂₄-Fettsäuren, und unter diesen C₆-C₁₈-Feftsäuren, und insbesondere C₈-C₁₈-Fettsäuren bevorzugt geeignet. Die Fettsäuren können verzweigt oder unverzweigt, gesättigt oder ungesättigt sein. Erfindungsgemäß bevorzugt ist die Verwendung bei 20 °C flüssiger Glyceride pflanzlicher Herkunft, insbesondere von Cocoglyceriden, einer Mischung aus vorwiegend Di- und Triglyceriden mit C₈-C₁₈-Fettsäuren, die beispielsweise unter der Bezeichnung Myritol®331 von der Cognis Deutschland GmbH vertrieben werden. Ebenso bevorzugt ist die Verwendung von Myritol® 312 (C₈/C₁₀-Trigiyceride). Cegesor® PS 17, Cegesoft® GPO; Cegesoft® PFO und Cegesoft® PS 6, die den Zusammensetzungen nach Applikation besonders gute pflegende Eigenschaften verleihen.

Als Ölkörper kommen auch bei 20° C flüssige Guerbetalkohole auf Basis von Fettalkoholen mit 6 bis 18, vorzugsweise 8 bis 10 Kohlenstoffatomen, wie beispielsweise Eutanol® G, in Frage. Auch flüssige Ester von linearen, gesättigten oder ungesättigten C₆-C₂₂-Fettsäuren mit linearen oder verzweigten, gesättigten oder ungesättigten C₆-C₂₂-Fettalkoholen bzw. Ester von verzweigten C₆-C₁₃-Carbonsäuren mit linearen oder verzweigten, gesättigten oder ungesättigten C₆-C₂₂-Fettalkoholen, wie beispielsweise Cetiol® CC, sind erfindungsgemäß als Ölkörper einsetzbar.

Unter den Wachsestem seien -expemplarisch folgende typische Vertreter genannt Decyloleat (Cetiol® V), Cococaprylatel-caprat (Cetiol® SN), Hexyllaurat (Cetiol® A), Myristyimyristat (Cetiol® MM), Myristylisostearat, Myristyloleat, Cetylisostearat, Cetyloleat, Stearylisostearat, Stearyloleat, Isostearylmyristat, Isostearylpalmitat, Isostearylstearat, Isostearylisostearat, Isostearyloleat, Isostearylbehenat, Isostearyfoleat, Oleylmyristat, Oleylpalmitat, Oleylstearat, Oleylisostearat, Oleyloleat (Cetiol®DAB), Oleylbehenat, Oleylerucat (Cetiol®J 600), Behenylisostearat, Behenyloleat, Erucylisostearat, Erucyloleat, Daneben eignen sich auch Ester von linearen C₆-C₂₂-Fettsäuren mit verzweigten Alkoholen, insbesondere 2-Ethylhexanol (Cetiol® 868), Ester von verzweigten C₆-C₂₂₋Fettsäuren mit linearen Alkoholen, Ester von C₁₈-C₃₈-Alkylhydroxycarbonsäuren mit linearen oder verzweigten C₆-C₂₂-Fettalkoholen, Ester von linearen und/oder verzweigten Fettsäuren mit mehrwertigen Alkoholen (wie z. B. Propylenglycol, Dimerdiol oder Trimertriol) und/oder Guerbetalkoholen, sowie Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen (z. B. Dioctyl Malate) oder Polyolen mit 2 bis 10 Kohlenstoffatomen und 2 bis 6 Hydroxylgruppen.

Zu den erfindungsgemäß einsetzbaren Ölkörpem zählen auch bei 20 °C flüssige natürliche und synthetische, aliphatische und/oder naphthenische Kohlenwasserstoffe, wie z. B. Squalan, Squalen, Paraffinöle, Isohexadecan, Isoeicosan oder Polydecene sowie Dialkylcyclohexane (Cetiol® S).

Erfindungsgemäß sind als Ölkörper auch flüssige Siliconöle geeignet. Zu diesen zählen z. B. Dialkyl-und Alkylarylsiloxane, wie beispielsweise Cyclomethicone, Dimethylpolysiloxan und Methylphenylpolysiloxan, sowie deren alkoxylierte und quaternierte Analoga. Geeignete nicht-flüchtige Siliconöle, wie z. B. Polyalkylsiloxane, Polyalkylarylsiloxane und Polyethersiloxan-Copolymere sind in **Cosmetics: Science and Technology, Hrsg.: M. Balsam und E. Sagarin, Bd. 1,1972, S. 27-104,** in **US 4,202,879** und **US 5,069,897** beschrieben. Eine bevorzugte Ausführungsform der erfindungsgemäßen Zusammensetzung enthält zusätzlich wenigstens eine Siliconverbindung ausgewählt aus der Gruppe der Siliconöle oder der Siliconwachse. Der Zusatz von Siliconverbindungen vermittelt ein besonders leichtes Hautgefühl.

### Wirkstoffe

Eine bevorzugte Ausführungsform der erfindungsgemäßen Zusammensetzung enthält zusätzlich wenigstens einen Wirkstoff. Die Wirkstoffe können erfindungsgemäß auch in verkapselter oder mikroverkapselter Form eingesetzt werden.
Unter Wirkstoffen werden erfindungsgemäß Stoffe verstanden, die zum Schutz der Haut und zur Stärkung der Hautbarriere beitragen, die reizlindemd, antimikrobiell oder hautbefeuchtend wirken. Erfindungsgemäß bevorzugt sind Wirkstoffe, die zur L-inderung entzündlicher Hautprozesse oder geröteter, wunder Haut dienen, zu denen beispielsweise auch Zinkverbindungen oder Schwefel zählen. Der Wirkstoff ist - je nach Art - üblicherweise in einer Menge von 0,01-10 Gew.-%, vorzugsweise 0,1-7 Gew.-% und insbesondere 1- 5 Gew.-% enthalten. Erfindungsgemäß bevorzugt sind öllösliche Wirkstoffe, obgleich sich durch Zugabe von Emulgatoren und/oder Solubilisatoren auch begrenzte Mengen wasserlöslicher Wirkstoffe einarbeiten lassen. Die Wirkstoffe können auch in beliebiger Kombination eingesetzt werden.

Geeignet sind z. B. auch Pflanzenextrakte, die häufig eine synergistisch wirkende Kombination wundheilender/reizlindernder Stoffe enthalten. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf **Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt,** beginnenden Tabelle aufgeführt sind.

Erfindungsgemäß sind vor allem die Extrakte aus Kamille, Aloe Vera, Hamamelis, Lindenblüten, Roßkastanie, Grünem Tee, Eichenrinde, Brennessel, Hopfen, Klettenwurzel, Schachtelhalm, Weißdorn, Mandel, Fichtennadel, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel geeignet.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit. Wasser, bevorzugt. Pflanzenextrakte auf Basis von WasserlPropylenglykol im Verhältnis 1:10 bis 10:1 haben sich als besonders geeignet erwiesen.

### Antimikrobielle/biogene Wirkstoffe

Typische Beispiele für **keimhemmende Mittel** sind Konservierungsmittel mit spezifischer Wirkung gegen gram-positive Bakterien wie etwa 2,4,4'-Trichlor 2'-hydroxydiphenylether, Chlorhexidin (1,6-Di-(4-chlorphenyl-biguanido)hexan) oder TCC (3,4,4'-Trichlorcartianilid). Auch zahlreiche Riechstoffe und etherische Öle weisen antimikrobielle Eigenschaften auf. Typische Beispiele sind die Wirkstoffe Eugenol, Menthol und Thymol in Nelken-, Minz- und Thymianöl. Ein interessantes natürliches Deomittel ist der Terpenalkohol Famesol (3,7,11-Trimethyl-2,6,10-dodecatrien-1-ol), der im Lindenblütenöl vorhanden ist und einen Maiglöckchengeruch hat, sowie das aus Schalentieren gewonnene Chitosan.

Auch Glycerinmonolaurat, Glycerinstearat, Glycerinoleat sowie Glycerindioleat haben sich als keimhemmend erwiesen und sind wegen ihrer außerordentlichen Milde und Unbedenklichkeit besonders im Bereich der Baby-Hygiene und -Pflege vorteilhaft einsetzbar. Unter biogenen **Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phyfantriol, Panthenol, α-Hydroxycarbonsäuren, Aminosäuren, Ceramide, Pseudoceramide, essentielle Öle, Pflanzenextrakte und Vitaminkomplexe zu verstehen. Erfindungsgemäß bevorzugt als Wirkstoffe sind öllösliche Vitamine und Vitaminvorstufen. Ganz besonders bevorzugt sind Tocopherol (Vitamin-E) und Tocopherol-Derivate.

Als Wirkstoffe sind erfindungsgemäß auch Verbindungen einsetzbar, die unter der Bezeichnung Generol® im Handel sind. Hierzu zählen ethoxylierte und nicht-ethoxylierte Phytosterine.

Üblicherweise liegt der Anteil der keimhemmenden Mittel bei etwa 0,1 bis 2 Gew.-% - bezogen auf die Gesamtzusammensetzung. Die Glycerinester sind in höheren Mengen einsetzbar (vide supra).

### Feuchthaltemittel/Hautbefeuchtungsmittel

In einer bevorzugten Ausführungsform enthält die erfindungsgemäße Zusammensetzung als Wirkstoff auch ein Feuchthaltemittel. Dieses dient zur Verbesserung der sensorischen Eigenschaften der Zusammensetzung sowie zur Feuchtigkeitsregulierung der Haut. Es kann außerdem dazu beitragen, das Eindringvermögen der Zusammensetzung auf den Wipes zu verbessern. Feuchthaltemittel sind üblicherweise in einer Menge von 0,1 - 20 Gew.-%, vorzugsweise 1-15 Gew.-%, und insbesondere 5 -10 Gew.-% enthalten.

Erfindungsgemäß geeignet sind u.a. Aminosäuren, Pyrrolidoncarbonsäure, Milchsäure und deren Salze, Lactitol, Harnstoff und Hamstoffderivate, Hamsäure, Glucosamin, Kreatinin, Spaltprodukte des Kollagens, Chitosan oder Chftosansalze/-derivate, und insbesondere Polyole und Polyolderivate (z. B. Glycerin, Diglycerin, Triglycerin, Ethylenglycol, Propylenglycol, Butylenglycol. Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin und Triglycerin.

### Emulgatoren

Eine weitere bevorzugte Ausführungsform der erfindungsgemäßen Zusammensetzung enthält zusätzlich wenigstens einen Emulgator. Durch den Zusatz von W/O und O/W-Emulgatoren lassen sich geringe Mengen wasserlöslicher Substanzen und Wirkstoffe, Wasser sowie Feuchthaltemittel einarbeiten.

Erfindungsgemäß bevorzugt sind nicht-ionische Emulgatoren. Diese zeichnen sich durch ihre Hautfreundlichkeit und Milde sowie ihre ökotoxologisch guten Eigenschaften aus. Durch Verwendung einer Kombination nicht-ionischer W/O und O/W-Emulgatoren erhält man Zusammensetzungen mit verbesserter Sensorik. Die erfindungsgemäßen Zusammensetzungen enthalten den/die Emulgator(en) in einer Menge von 0 bis 20 Gew.-%, vorzugsweise 0,1 bis 15 Gew.-% und insbesondere 0,1 bis 10 Gew.-% bezogen auf das Gesamtgewicht der Zusammensetzung.

### Nicht-ionische Emulgatoren

Zur Gruppe der nicht-ionischen Emulgatoren gehören beispielsweise:
(1) Anlagerungsprodukte von 2 bis 50 Mol Ethylenoxid und/oder 1 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.
(2) C₁₂-C₁₈-Fettsäuremono- und -diester von Anlagerungsprodukten von 1 bis 50 Mol Ethylenoxid an Glycerin.
(3) Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z. B. Polyolpoly-12-hydroxystearate, Polyglycerinpolyricinoleat, Polyglycerindiisostearat oder Polyglycerindimerat. Ebenfalls geeignet sind Gemische von Verbindungen aus mehreren dieser Substanzklassen.
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆-C₂₂-Fettsäuren, Ricinolsäure sowie 12-Hydroxystearinsäure und Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose), oder Mischester wie z. B. Glycerylstearatcitrat und Glycerylstearatlactat.
(9) Polysiloxan-Polyalkyl-Polyether-Copolymere bzw. entsprechende Derivate.
(10) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. C_{12/18}-Fettsäuremono-und -diester von Anlagerungsprodukten von. Ethylenoxid an Glycerin sind als Rückfettungsmittel für kosmetische Zubereitungen bekannt.

Erfindungsgemäß besonders gut geeignete und milde Emulgatoren sind Polyolpoly-12-hydroxystearate und Abmischungen davon, welche beispielsweise unter den Marken "Dehymuls® PGPH" (W/O-Emulgator) oder "Eumulgin® VL 75" (Abmischung mit Coco Glucosides im Gewichtsverhältnis 1:1, O/W-Emulgator) oder Dehymuls® SBL (W/O-Emulgator) von der Cognis Deutschland GmbH vertrieben werden. In diesem Zusammenhang sei insbesondere auf das Europäische Patent EP 0 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Als lipophile W/O-Emulgatoren eignen sich prinzipiell Emulgatoren mit einem HLB-Wert von 1 bis 8, die in zahlreichen Tabellenwerken zusammengefaßt und dem Fachmann bekannt sind. Einige dieser Emulgatoren sind beispielsweise in **Kirk-Othmer, "Encyclopedia of Chemical Technology",** 3. Aufl., **1979, Band 8, Seite 913,** aufgelistet. Für ethoxylierte Produkte läßt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von C₄-C₆-Polyolen, wie beispielsweise Partialester des Pentaerythrits oder Zuckerestern, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbüantrierucat, Sorbitanrnonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Im Falle der Einarbeitung wasserlöslicher Wirkstoffe und/oder geringer Mengen Wasser kann es weiterhin vorteilhaft sein, zusätzlich wenigstens einen Emulgator aus der Gruppe nicht-ionischer O/W-Emulgatoren (HLB-Wert: 8 - 18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10 - 20 Ethylenoxid-Einheiten für O/W-Emulgatoren und 20 - 40 Ethylenoxid-Einheiten für sogenannte Solubilisatoren. Erfindungsgemäß besonders vorteilhaft als O/W-Emulgatoren sind Ceteareth-12 und PEG-20 Stearat. Als Solubilisatoren bevorzugt geeignet sind Eumulgin® HRE 40 (INCI: PEG-40 Hydrogenated Castor Oil), Eumulgin® HRE 60 (INCI: PEG-60 Hydrogenated Castor Oil), Eumulgin® L (INCI: PPG-1-PEG-9 Laurylglycolether), sowie Eumulgin® SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. C₈-C₂₂-Alkylmono- und oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, daß sowohl Monoglycoside, bei denen ein cydischer Zuckeffest glycosidisch an den Fettalkohol gebunden ist, als auch ollgomere Glycoside mit einem Oigomensationsgrad bis vorzugsweise etwa 8 geeignet sind. Der Oligomensierungsgrad ist dabei ein statistischer Mittelwert, dem eine für solche technischen Produkte übliche Homologenverteilung zugrunde liegt Produkte, die unter der Bezeichnung Plantacare® zur Verfügung stehen, enthalten eine glucosidisch gebundene C₈-C₁₆-Alkylgruppe an einem Oligoglucosldrest, dessen mittlerer 0ligomertsationsgrad bei 1 bis 2 liegt. Auch die vom Glucamin abgeleiteten Acylglucamide sind als nicht-ionische Emulgatoren geeignet. Erfindungsgemäß bevorzugt Ist ein Produkt, das unter der Bezeichnung Emulgade® PL 68/50 von der Cognis Deutschland GmbH vertrieben und ein 1:1-Gemisch aus Alkylpolyglucosiden und Fettalkoholen darstellt Erfindungsgemäß vortellhaft einsetzbar ist auch ein Gemisch aus Lauryl Glucoside, Polyglyceryl-2-Dipolyhydroxystearate, Glycerin und Wasser, das unter der Bezeichnung Eumulgin® VL 75 im Handel ist

Eine besonders bevorzugte Ausführungsform der erfindungsgemäßen Zusammensetzung enthält
(a) 1-50 Gew.-% wenigste einer Öl-oder Wachskomponents ausgewählt aus C₁₄-C₃₀-Dialkyl(en)carbonaten und gegebenenfalls zusätzlich eine Öl- oder wacnhskomponente ausgewäult aus C₁₄-C₃₀-Dialkyl(en)carbonaten C₉-C₃₄-Dicarbonsäuren oder C₁₂-C₃₀-Hydroxyfettalkoholen oder einem beliebigen Gemisch ethern dieser Substanzen,
(b) 0,1- 5 Gew.-% wenigstens eines Wirkstoffes,
(c) 1-10 Gew.-% wenigstens eines Öls,
(d) 0,1-10 Gew.-% wenigstens eines Emulgators,
(e) 5 - 90 Gew.-% weiterer Wachskomponenten und
(f) 0-5 Gew.-% Wasser.

### Weitere Tenside/Emulgatoren (fakultativ)

Die Zusammensetzungen können je nach Verwendungszweck der Wipes und Tissues weiterhin zwitterionische, amphotere, kationische und ferner anionische Tenside enthalten. Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind ampholytische Tenside. Unter ampholytischen Tensiden werden solche oberflächenaktiven Verbindungen verstanden, die außer einer C₈-C₁₈-Alkyl-oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N- Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Anionische Tenside sind gekennzeichnet durch eine wasserlöslich machende, anionische Gruppe wie z. B. eine Carboxylat-, Sulfat-, Sulfonat- oder Phosphat-Gruppe und einen lipophilen Rest Hautverträgliche anionische Tenside sind dem Fachmann in großer Zahl aus einschlägigen Handbüchem bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Als kationische Tenside sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex® vertriebenen Dialkylammoniummethosulfate und Methylhydroxyalkyldialkoyloxyalkylammoniummethosulfate und die entsprechenden Produkte der Dehyquart®-Reihe, als kationische Tenside eingesetzt werden. Unter der Bezeichnung "Esterquats" werden im allgemeinen quaternierte Fettsäuretriethanolaminestersalze verstanden. Sie können den erfindungsgemäßen Zusammensetzungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

### Weitere Hilfs- und Zusatzstoffe

Die erfindungsgemäßen Zusammensetzungen können je nach Art und Zweck der Applikation eine Reihe weiterer Hilfs- und Zusatzstoffe enthalten, wie beispielsweise Überfettungsmittel, Verdickungsmittel, Polymere, Wachse, biogene Wirkstoffe, Deowirkstoffe, Filmbildner, UV-Lichtschutzfaktoren, Antioxidantien, Hydrotrope, Konservierungsmittel, Insektenrepellentien, Selbstbräuner, Solubilisatoren, Parfümöle, Farbstoffe, und dergleichen.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden.

Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Anorganische und organische Puder tragen zur weiteren Verbesserung der sensorischen Eigenschaften des Produktes bei. Eine bevorzugte Ausführungsform der erfindungsgemäßen Zusammensetzung enthält daher weiterhin wenigstens einen Puderstoff. Dieser ist üblicherweise in einer Menge von 0,5 -10 Gew.-%, vorzugsweise 1 - 8 Gew.-% und ganz besonders bevorzugt 1 - 5 Gew.-% bezogen auf die Gesamtzusammensetzung in den erfindungsgemäßen Zusammensetzungen enthalten. Unter Pudern wird im allgemeinen eine Anhäufung von Festteilchen mit einer Teilchengröße unter 100 nm verstanden, die als medizinisches oder kosmetisches Präparat zur lokalen Anwendung auf der gesunden oder kranken Haut dient (**Quelle: CD Römpp Chemie Lexikon - Version 1.0, Stuttgart/New York: Georg Thieme Verlag 1995**). Man unterscheidet bezüglich der Konsistenz flüssige Puder, lose Puder (Streupuder), festgepreßte Puder (Compacts), Puder-Crèmes sowie Puder in Aerosolform. All diese Formen lassen sich in die erfindungsgemäßen Mittel einarbeiten. Als Hauptbestandteil der Puder kommen feinpulvrige, einfache od. gemischte, saugfähige, gut deckende, an der Haut haftende, ungiftige Stoffe in Frage, wie Siliciumdioxid, gefällte Kreide, Magnesiumcarbonat, Kaolin, Talk, Zinkoxid, Titandioxid, Strontiumcarbonat u. -sulfat, Calciumsulfat, Bismutsalze, Stearate von Mg, Zn, Ti, Ca u. Al, ferner Reis-, Mais- u. Weizenstärke, Lycopodium, lriswurzel, und gemahlene Seide. Besonders bevorzugt sind erfindungsgemäß Aluminium Starch Octenylsuccinate (Dry Flo® PC), Talk, "Babypuder" und Silicagel.

Geeignete **kationische Polymere** sind beispielsweise kationische Cellulosederivate, wie z. B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinylimidazol-Polymere, wie z.B. Luviquat® (BASF), Kondensationsprodukte von Polyglycolen und Aminen, quatemierte Kollagenpolypeptide, wie beispielsweise Lauryldimonium hydroxypropyl hydrolyzed collagen (Lamequat®L/Grünau), quatemierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z. B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, VinylpyrrolidonNinylacrylat-Copolymere, Vinylacetat/Butylmaleat/ Isobornylacrylat-Copolymere, MethylvinyletherlMaleinsäureanhydrid-Copolymere und deren Ester, unvernetzte und mit Polyolen vernetzte Polyacrylsäuren, Acrylamidopropyltrimethylammoniurnchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, VinylpyrrolidonNinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Für Applikationen der erfindungsgemäßen Zusammensetzungen auf Tissues und Wipes, die zur Reduktion des Körpergeruchs und der Schweißbildung dienen, werden zusätzlich Deo- und Antitranspirant-Wirkstoffe eingearbeitet. Hierfür kommen z. B. Antitranspirantien wie etwa Aluminiumchlorhydrate, Aluminium-Zirkonium-Chlorohydrate sowie Zinksalze in Frage. Diese wirken wahrscheinlich über den partiellen Verschluß der Schweißdrüsen durch Eiweiß- und/oder Polysaccharidfällung. Neben den Chlorhydraten können auch Aluminiumhydroxylactate sowie saure Aluminium/Zirkoniumsalze eingesetzt werden. Unter der Marke Locron® der Clariant GmbH, befindet beispielsweise sich ein Aluminiumchlorhydrat im Handel, das der Formel [Al₂(OH)₅Cl]·2,5 H₂O entspricht, und dessen Einsatz besonders bevorzugt ist. Ebenso erfindungsgemäß bevorzugt ist der Einsatz von Aluminium-Zirkonium-Tetrachlorohydrex-Glycin-Komplexen, die beispielsweise von Reheis unter der Bezeichnung Rezal® 36G vermarktet werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® C.A.T., Cognis Deutschland GmbH). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw. -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester. Antibakterielle Wirkstoffe, welche die Keimflora beeinflussen und schweißzersetzende Bakterien abtöten bzw. in ihrem Wachstum hemmen, können ebenfalls in den Zusammensetzungen enthalten sein. Beispiele hierfür sind Chitosan, Phenoxyethanol und Chlorhexidingluconat. Besonders wirkungsvoll hat sich auch 5-Chlor-2-(2,4-dichlorphenoxy)-phenol erwiesen, das unter der Bezeichnung Irgasan® von der Ciba-Geigy, Basel/CH vertrieben wird.

Wird die erfindungsgemäße Zusammensetzung auf Wipes zum Sonnenschutz appliziert, so werden zusätzlich Lichtschutzfaktoren eingearbeitet. Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UVB-Filter können öllöslich oder wasserlöslich sein. Als öllösliche Substanzen, die erfindungsgemäß bevorzugt sind, sind z. B. zu nennen:
- 3-Benzylidencampher bzw. 3-Benzylidennorcampher und dessen Derivate, z. B. 3-(4-Methyl-benzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dhydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon;
- Propan-1,3-dione, wie z.B. 1-(4-tert.Butylphenyl)-3-(4'methoxyphenyl)propan-1,3-dion;
- Ketotricyclo(5.2.1.0)decan-Derivate;

Als wasserlösliche UV-Filter-Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa)-oder Eusolex® T 2000 (Merck).-Als hydrophobe- Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt, z. B. mikronisiertes Zinkoxid.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z. B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Superoxid-Dismutase, Zink und dessen Derivate (z. B. ZnO, ZnSO₄) Selen und dessen Derivate (z. B. Selen-Methionin), Stilbene und deren Derivate (z. B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Einige dieser Verbindungen wurden bereits unter den Feuchthaltemittein erwähnt. Typische Beispiele sind:
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als Selbstbräuner eignet sich Dihydroxyaceton.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzem (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcydohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, lrotyl und Floramat allein oder in Mischungen, eingesetzt.

Als **Farbstoffe** können die für kosmetische Zwecke geeigneten und zugelassenen Substanzen verwendet werden. Die Farbstoffe können öllöslich oder wasserlöslich sein und werden üblicherweise in Konzentrationen von 0,001 bis 0,1 Gew.-%, bezogen auf die gesamte Mischung, eingesetzt. In einer bevorzugten Ausführungsform enthalten die erfindungsgemäßen Zusammensetzungen wenigstens einen öllöslichen Farbstoff. Der Zusatz des Farbstoffs hat den Vorteil, daß die Stabilität der auf die Wipes applizierten Zusammensetzung leicht visualisierbar ist. Beispielsweise kann so bei einer wässrig/tensidischen Nachbehandlung der Wipes kontrolliert werden, ob sich Wachs- und Wasserphase bei längerer Lagerung allmählich vermischen. Als öllösliche Farbstoffe eignen sich beispielsweise C. I. 47000, C. I, 67565, C. I. 26100, C. I. 60725, C. I. 12150, C. I. 75810, C. I. 75300.

### Formullerungsbeisplele

Zur Prüfung der anwendungstechnischen Eigenschaften wurde die Stabilität der erfindungsgemäßen Zusammensetzung überprüft und die Sensorik im Probandentest beurteilt. Handetsübliche Wipes (Substrat) mit einem Gewicht von 60 g/m² wurden mit den erfindungsgemäßen Zusammensetzungen 1 bis 11 sowie den Vergleichszubereitungen V1 und V2 in Mengen von jeweils 0,3 g pro Gramm Substrat 195 g/m² beschichtet. Die mit den erfindungsgemäßen Zusammensetzungen beschichteten Wipes sind bezüglich Sensorik und Lagerstabilität den herkömmllchen Zusammensetzungen des Standes der Technik überfegen. Die lagerstabilität der Wipes wurde, nachdem die Zusammensetzungen zusammen mit einem Farbstoff auf die Wipes appliziert (Phase 1) wurden, und die Wipes einer Nachbehandlung mit einer wässrig/tensidischen Lösung (Phase 2) unterzogen wurden, nach 12-wöchiger Lagerung beurteilt. Durch den Farbstoff ist eine eventuelle Vermischung der Phasen leicht erkennbar.

Die Mengenangaben in nachfolgenden Beispielen beziehen sich, soweit nicht anders angegeben, auf Gew.-% der handelsüblichen Substanzen in der Gesamtzusammensetzung. Die Beispiele 1 bis 11 sind erfindungsgemäße Formulierungen, V1 und V2 stellen Vergleichsbeispiele dar.

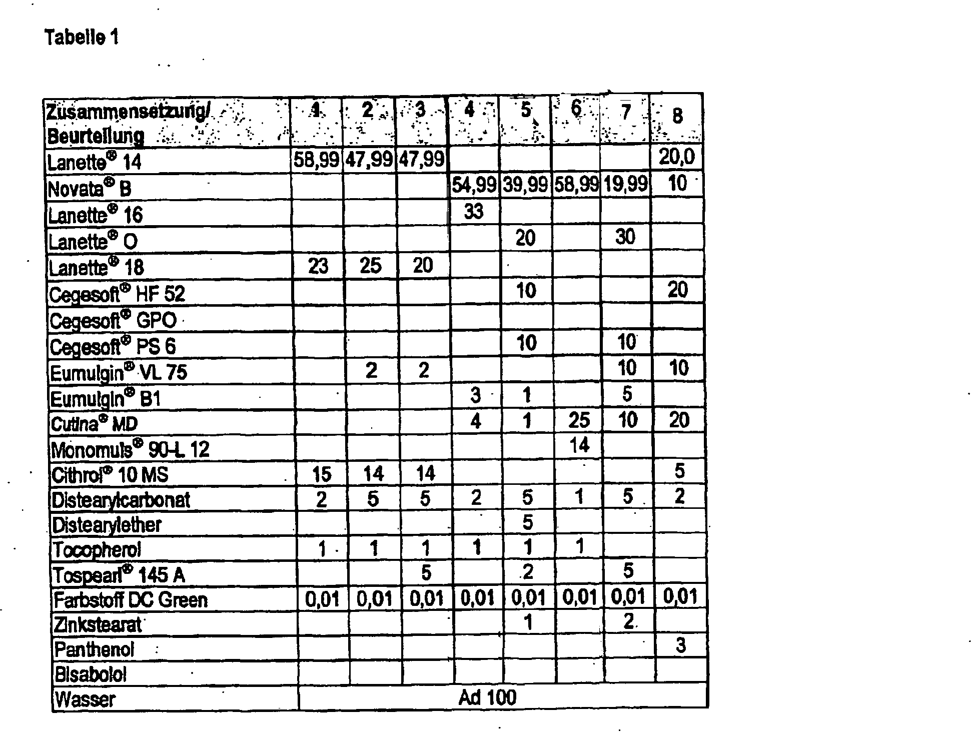

Die Penetraäonswerte gemessen mit dem Penetrometer (Petrotester PNR 10, Mikrotconus, 5sec., 20 °C) betragen 0,54mm - 2,43mm

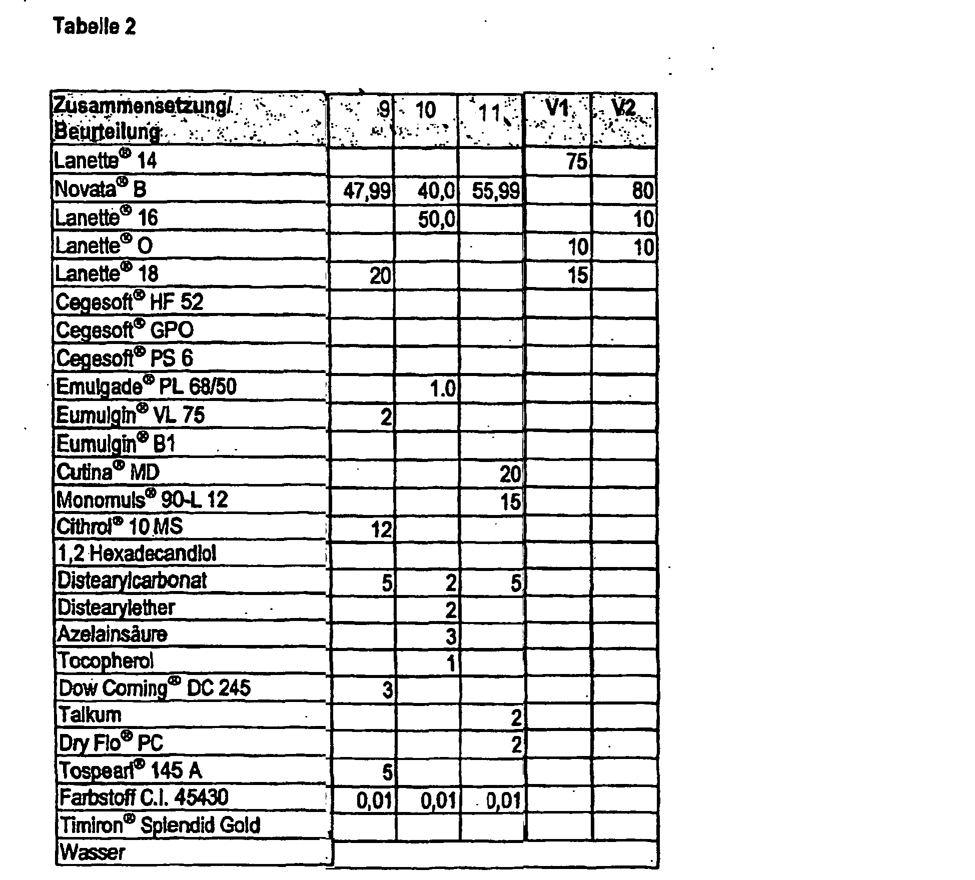

Die Penetrationswerte gemessen mit dem Penetrometer (Petrotester PNR 10, Mikrokonus, 5sec., 20 °C) betragen 0,54mm - 2,43mm

### Anhang

1) Cegesoft® GPO
   INCI: Palm (Elaeis Guineensis) Oil
   Hersteller: Cognis Deutschland GmbH (Henkel)
2) Cegesoft® HF 52
   INCl: Hydrogenated Vegetable Oil
   Hersteller: Cognis Deutschland GmbH (Henkel)
3) Cegesoft® PS 6
   INCl: Vegetable Oil
   Hersteller: Cognis Deutschland GmbH (Henkel)
4) Cithrol® 10 MS
   INCI: PEG-20 Stearate
   Hersteller: Croda Surfactants Ltd.
5) Cutina® MD
   INCI: Glyceryl Stearate
   Hersteller: Cognis Deutschland GmbH (Henkel)
6) Dow Coming DC® 245
   INCI: Cyclomethicone
   Hersteller Dow Coming
7) Dry Flo® PC
   INCI: Aluminum Starch Octenylsuccinate
   Hersteller: National Starch
8) Emulgade® PL 68/50
   lNCl: Cetearyl Glucoside, Cetearyl Alcohol
   Hersteller. Cognis Deutschland GmbH (Henkel)
9) Eumulgin ® B1
   INCI: Ceteareth-12
   Hersteller: Cognis Deutschland GmbH (Henkel)
10) Eumlugin® VL 75
   lNCl: Lauryl Glucoside, Polyglyceryl-2 Dipolyhydroxystearate, Glycerin, Aqua (Water); ca. 75 % Aktivsubstanz in Wasser
   Hersteller: Cognis Deutschland GmbH (Henkel)
11) Lanette® 14
   INCI: Myristyl Alcohol
   Hersteller: Cognis Deutschland GmbH (Henkel)
12) Lanette® 16
   INCl: Cetyl Alcohol
   Hersteller: Cognis Deutschland GmbH (Henkel)
13) Lanette® 18
   INCI: Stearyl Alcohol
   Herstellen: Cognis Deutschland GmbH (Henkel)
14) Lanette® O
   INCI: Cetearyl Alcohol
   Hersteller: Cognis Deutschland GmbH (Henkel)
15) Monomuls® 90-L 12
   INCI: Glyceryl Laurate
   Hersteller Cognis Deutschland GmbH (Henkel)
16) Novata® B
   INCI: Cocoglycerides
   Hersteller. Cognis Deutschland GmbH (Henkel)
17) Vitamin E Primaspheres®
   INCI: Vitamin E
   Hersteller: Primacare; Cognis Iberia
18) Vitamin A Primaspheres®
   INCI: Vitamin A
   Hersteller: Primacare; Cognis Iberia
19) Timiron® Splendid Gold
   INCI: Titanium Dioxide and Mica and Silica
   Hersteller: Rona EM Industries, Inc. NY
20) Tospearl® 145 A
   INCI: Polymethylsilsesquioxane
   Hersteller: Bayer GE Silicones
21) Tospearl® 145 A
   lNCl: Polymethylsihesquioxane
   Hersteller: Bayer GE Silicones

## Patentansprüche

1. Zusammensetzung auf Wachsbasis mit einem Schmelzpunkt oberhalb 25 °C enthaltend
(a) wenigstens eine ÖI- oder Wachskomponente ausgewählt aus den C₁₄-C₃₀-Dialkyl(en)carbonaten und
(b) weniger als 10 Gew.-% Wasser.

2. Zusammensetzung gemäß Anspruch 1, **dadurch gekennzeichnet, daß** sie zusätzlich Dialkyl(en)ether, Dicarbonsäuren oder Hydroxyfettalkohole oder ein beliebiges Gemisch dieser /Substanzen enthält.

3. Zusammensetzung gemäß einem der Ansprüche 1 oder 2, **dadurch gekennzeichnet, dass** sie im Bereich von ca. 30 - 45°C schmilzt.

4. Zusammensetzung gemäß einem der Ansprüche 1 bis 3, **dadurch gekennzeichnet, daß** der Wassergehalt weniger als 6 Gew.-%, vorzugsweise weniger als 3 Gew.%, beträgt.

5. Zusammensetzung gemäß einem der Ansprüche 1 bis 4, **dadurch gekennzeichnet, daß** sie wenigstens eine weitere wachsartige Lipidkomponente enthält.

6. Zusammensetzung gemäß Anspruch 5, **dadurch gekennzeichnet, daß** als weitere wachsartige Lipidkomponente wenigstens ein Fettalkohol und/oder Fettsäureglycerid enthalten ist.

7. Zusammensetzung gemäß einem der Ansprüche 1 bis 6, **dadurch gekennzeichnet, daß** sie zusätzlich wenigstens einen weiteren Ölkörper enthält.

8. Zusammensetzung gemäß einem der Ansprüche 1 bis 7, **dadurch gekennzeichnet, daß** sie zusätzlich wenigstens eine Siliconverbindung ausgewählt aus der Gruppe der Siliconöle oder der Siliconwachse enthält.

9. Zusammensetzung gemäß einem der Ansprüche 1 bis 8, **dadurch gekennzeichnet, daß** sie zusätzlich wenigstens einen Emulgator enthält.

10. Zusammensetzung gemäß einem der Ansprüche 1 bis 9, **dadurch gekennzeichnet, daß** sie zusätzlich einen Wirkstoff enthält.

11. Zusammensetzung gemäß einem der Ansprüche 1 bis 10, **dadurch gekennzeichnet, daß** sie zusätzlich wenigstens einen Puderstoff enthält.

12. Zusammensetzung gemäß einem der Ansprüche 1 bis 11, **dadurch gekennzeichnet, daß** sie mit dem Petrotester PNR 10 unter Verwendung eines Mikrokonus bei 5 Sekunden Messdauer bei 20 °C Penetrationswerte von 0,2 - 3 mm aufweist.

13. Zusammensetzung gemäß einem der Ansprüche 1 bis 12, **dadurch gekennzeichnet, daß** sie
(a) 1 - 50 Gew.-% wenigstens einer Öl- oder Wachskomponente ausgewählt aus den C₁₄-C₃₀-Dialkyl(en)carbonaten,
(b) 0,1 - 5 Gew.-% wenigstens eines Wirkstoffes,
(c) 1 - 10 Gew.-% wenigstens eines Öls,
(d) 0,1 - 10 Gew.-% wenigstens eines Emulgators,
(e) 5 - 90 Gew.-% weiterer Wachskomponenten und
(f) 0 - 5 Gew.-% Wasser
enthält.

14. Zusammensetzung gemäß einem der Ansprüche 1 bis 13, **dadurch gekennzeichnet, daß** sie wenigstens einen öllöslichen Farbstoff enthält.

15. Zusammensetzung gemäß einem der Ansprüche 1 bis 14, **dadurch gekennzeichnet, daß** sie wenigstens ein Feuchtemittel enthält.

16. Verwendung einer Zusammensetzung gemäß einem der Ansprüche 1 bis 15 als Körperpflegemittel.

## Claims

1. Wax-based composition with a melting point above 25°C containing
(a) at least one oil or wax component selected from C₁₄₋₃₀ dialkyl(ene) carbonates and
(b) less than 10% by weight water.

2. A composition as claimed in claim 1, **characterized in that** it additionally contains dialkyl(ene) ethers, dicarboxylic acids or hydroxyfatty alcohols or a mixture of these substances.

3. A composition as claimed in claim 1 or 2, **characterized in that** it melts at about 30 to 45°C.

4. A composition as claimed in any of claims 1 to 3, **characterized in that** the water content is less than 6% by weight and preferably less than 3% by weight.

5. A composition as claimed in any of claims 1 to 4, **characterized in that** it contains at least one other wax-like lipid component.

6. A composition as claimed in claim 5, **characterized in that** it contains at least one fatty alcohol and/or fatty acid glyceride as the other wax-like lipid component.

7. A composition as claimed in any of claims 1 to 6, **characterized in that** it additionally contains at least one other oil component.

8. A composition as claimed in any of claims 1 to 7, **characterized in that** it additionally contains at least one silicone compound selected from the group of silicone oils or silicone waxes.

9. A composition as claimed in any of claims 1 to 8. **characterized in that** it additionally contains at least one emulsifier.

10. A composition as claimed in any of claims 1 to 9, **characterized in that** it additionally contains an active component.

11. A composition as claimed in any of claims 1 to 10, **characterized in that** it additionally contains at least one powder.

12. A composition as claimed in any of claims 1 to 11, **characterized in that** it has penetration values of 0.2 to 3 mm, as measured for 5 seconds at 20°C with a PNR 10 Petrotester using a microcone.

13. A composition as claimed in any of claims 1 to 12, **characterized in that** it contains
(a) 1 to 50% by weight of at least one oil or wax component selected from C₁₄₋₃₀ dialkyl(ene) carbonates,
(b) 0.1 to 5% by weight of at least one active component,
(c) 1 to 10% by weight of at least one oil,
(d) 0.1 to 10% by weight of at least one emulsifier,
(e) 5 to 90% by weight of other wax components and
(f) 0 to 5% by weight of water.

14. A composition as claimed in any of claims 1 to 13, **characterized in that** it contains at least one oil-soluble dye.

15. A composition as claimed in any of claims 1 to 14, **characterized in that** it contains at least one moisturizer.

16. The use of the composition claimed in any of claims 1 to 15 as a body care preparation.

## Revendications

1. Composition à base de cire ayant un point de fusion supérieur à 25°C, contenant :
(a) au moins un composant huileux ou cireux choisi parmi les carbonates de diakyl(èn)e en C₁₄ à C₃₀ et
(b) moins de 10 % en poids d'eau.

2. Composition selon la revendication 1,
**caractérisée en ce qu'**elle contient en outre
des éthers, de dialky(èn)e, des acides dicarboxyliques ou des alcools gras hydroxylès ou un mélange quelconque de ces substances.

3. Composition selon l'une des revendications 1 ou 2,
**caractérisée en ce qu'**
elle fond dans un domaine de température d'environ 30 à 45°C.

4. Composition selon l'une des revendications 1 à 3,
**caractérisée en ce que**
sa teneur en eau est inférieure à 6 % en poids, de préférence inférieure à 3 % en poids.

5. Composition selon l'une des revendications 1 à 4,
**caractérisée en ce qu'**
elle contient au moins un autre composant lipidique cireux.

6. Composition selon la revendication 5,
**caractérisée en ce que**
comme autre composant lipidique cireux, elle contient au moins un alcool gras et/ou un glycéride d'acide gras.

7. Composition selon l'une des revendications 1 à 6,
**caractérisée en ce que**
elle contient en outre, au moins un autre corps huileux.

8. Composition selon l'une des revendications 1 à 7,
**caractérisée en ce qu'**
elle contient en outre au moins un composé de silicone choisi dans le groupe des huiles de silicone ou des cires de silicone.

9. Composition selon l'une des revendications 1 à 8,
**caractérisée en ce qu'**
elle contient en outre au moins un émulsifiant.

10. Composition selon l'une des revendications 1 à 9,
**caractérisée en ce qu'**
elle contient en outre un principe actif.

11. Composition selon l'une des revendications 1 à 10,
**caractérisée en ce qu'**
elle contient en outre au moins une substance pulvérulente.

12. Composition selon au moins l'une des revendications 1 à 11,
**caractérisée en ce qu'**
elle présente des valeurs de pénétration à 20°C et pour une durée de mesure de 5 secondes de 0,2 à 3 mm avec le Petrotester PNR 10 en utilisant un micro-cône.

13. Composition selon l'une des revendications 1 à 12,
**caractérisée en ce qu'**
elle contient
(a) 1 à 50 % en poids d'au moins un composant huileux ou cireux choisi parmi les carbonates de diakyl(èn)e en C 14 à C30,
(b) 0,1 à 5 % en poids d'au moins un principe actif,
(c) 1 à 10 % en poids d'au moins une huile,
(d) 0,1 à 10 % en poids d'au moins un émulsifiant,
(e) 5 à 90 % en poids d'autres composants cireux et
(f) 0 à 5 % en poids d'eau.

14. Composition selon l'une des revendications 1 à 13,
**caractérisée en ce qu'**
elle contient au moins un colorant soluble dans l'huile.

15. Composition selon l'une des revendications 1 à 14,
**caractérisée en ce qu'**
elle contient au moins un humidifiant.

16. Utilisation d'une composition selon l'une des revendications 1 à 15, en tant que produit de soin pour le corps.
